# EUROPEAN PATENT SPECIFICATION

(11) **EP 2 937 110 B1**
(45) Date of publication and mention of the grant of the patent: **08.08.2018**
(21) Application number: 13866200.2
(22) Date of filing: 16.12.2013
(51) Int. Cl.: A61M 25/14, A61M 25/092, A61M 25/01, A61M 25/00

(54) **MEDICAL DEVICE**
MEDIZINISCHE VORRICHTUNG
DISPOSITIF MÉDICAL

(30) Priority: 18.12.2012 JP 2012275990
(43) Date of publication of application: 28.10.2015
(73) Proprietor: Sumitomo Bakelite Co., Ltd., Shinagawa-ku Tokyo 140-0002 (JP)
(72) Inventor: KANEMASA, Kenichi, Akita-shi Akita 011-8510 (JP); FUJITA, Yasuhiro, Akita-shi Akita 011-8510 (JP); YAMAGUCHI, Kenjiro, Akita-shi Akita 011-8510 (JP)
(74) Representative: Müller-Boré & Partner Patentanwälte PartG mbB
(86) International application number: PCT/JP2013/083591
(87) International publication number: WO 2014/098020

(56) References cited:
- WO-A1-2011/033783
- WO-A2-2006/065949
- JP-A- H09 266 949
- JP-A- 2011 083 594
- JP-A- 2011 251 068
- US-A1- 2007 173 757
- US-A1- 2008 091 169
- US-A1- 2012 277 671

## Description

### Technical Field

The present invention relates to a medical device.

Priority is claimed on Japanese Patent Application No. 2012-275990, filed December 18, 2012.

### Background Art

A medical device such as a catheter is known which is elongated and flexible, and is used by being inserted into a body cavity.

PTL 1 discloses a catheter which is configured so as to enable a bending operation of a distal portion by pulling an operation wire (tensile member in PTL 1) which is provided inside. The torsional rigidity of the catheter of PTL 1 is increased by a blade layer (mesh in PTL1) which is embedded in a resin layer. The operation wire is disposed further on the radially outside of the catheter than the blade layer.

PTL 2 discloses a catheter which has an inner coil with a small diameter and an outer coil with a large diameter, and in which an operation wire (control wire in PTL 2) is disposed between the inner coil and the outer coil.

### Citation List

### Patent Literature

[PTL 1] United States Patent Application, Publication No. 2010-0168827 (Japanese Unexamined Patent Application, First Publication No. 2010-155081)
[PTL 2] Published Japanese Translation No. 2007-503914 of the PCT International Publication for Patent

Other relevant medical devices can be found in documents US-A-2012/277671, WO-A-2011/033783 or US-A-2007/173757

### Summary of Invention

### Technical Problem

In the technique disclosed in PTL 1, the blade layer is only disposed further on the radially inside of the catheter than the operation wire. In this structure, there is room for improvement in terms of reinforcement around the operation wire.

In contrast, in the technique disclosed in PTL 2, the operation wire is inserted into a space, which has a crescent shape in section, between the outer coil and the inner coil by making the outer coil and the inner coil relatively eccentric. For this reason, the operation wire is lodged between the outer coil and the inner coil, and therefore, there is a possibility that the operation wire cannot smoothly slide with respect to the outer coil and the inner coil.

The present invention has been made in consideration of the above-described problems, and provides a medical device such as a catheter, which has favorable operation wire operability, and has a sufficiently reinforced structure around the operation wire.

### Solution to Problem

The present invention provides a medical device, as described in the claims, including:
an elongated resin medical device body which is flexible and is inserted into a body cavity;
a sub-lumen which is disposed inside the medical device body so as to extend along a longitudinal direction of the medical device body;
an operation wire which is inserted into the sub-lumen and extends along the longitudinal direction of the medical device body, and in which the medical device body is bent through a pulling operation with respect to the operation wire; and
a first tubular reinforcing layer and a second tubular reinforcing layer which are embedded in the medical device body such that axial directions of the reinforcing layers extend along an axial direction of the medical device body, to reinforce the medical device body,
in which the second reinforcing layer is disposed apart from the first reinforcing layer so as to surround the periphery of the first reinforcing layer,
in which the space between the first reinforcing layer and the second reinforcing layer is filled with a part of a resin constituting the medical device body, and
in which the sub-lumen is disposed inside the part of the resin.

According to the medical device, the second reinforcing layer is disposed apart from the first reinforcing layer so as to surround the periphery of the first reinforcing layer, and the space between the first reinforcing layer and the second reinforcing layer is filled with a part of a resin constituting the medical device body. The sub-lumen is disposed inside the part of the resin. The operation wire is inserted into the sub-lumen and extends along the longitudinal direction of the medical device body. Since the operation wire is inserted into the sub-lumen, it is possible to easily move the operation wire in the longitudinal direction of the sub-lumen relative to the sub-lumen, by sliding with respect to the peripheral wall of the sub-lumen. Moreover, the sub-lumen and the operation wire are protected by the first reinforcing layer positioned inside and the second reinforcing layer positioned outside. Accordingly, the peripheries of the sub-lumen and the operation wire are sufficiently reinforced.

That is, it is possible to provide the medical device that has a configuration in which the operability of the operation wire is good and the periphery of the operation wire is sufficiently reinforced.

### Advantageous Effects of Invention

According to the present invention, it is possible to provide a medical device that has a configuration in which the operability of an operation wire is good and the periphery of the operation wire is sufficiently reinforced.

### Brief Description of Drawings

FIG. 1 is a longitudinal cross-sectional view schematically showing a distal portion of a catheter as a medical device according to an embodiment.
FIG. 2 is a horizontal cross-sectional view schematically showing the catheter taken along B-B line of FIG. 1.
FIG. 3 is a horizontal cross-sectional view schematically showing the catheter taken along C-C line of FIG. 1.
FIG. 4 is a horizontal cross-sectional view schematically showing the catheter taken along D-D line of FIG. 1.
FIG. 5 is a horizontal cross-sectional view schematically showing the catheter taken along E-E line of FIG. 1.
FIG. 6 is a plan view schematically showing a distal portion of the catheter and a portion in the vicinity thereof.
FIG. 7a is a plan view schematically showing the catheter according to the embodiment.
FIG. 7b is a plan view schematically showing the catheter according to the embodiment.
FIG. 7c is a plan view schematically showing the catheter according to the embodiment.
FIG. 8 is a schematic view for illustrating an example of an operation of the catheter according to the embodiment.

### Description of Embodiments

Hereinafter, an embodiment of the present invention will be described using the drawings, but the present invention is not limited to the following embodiment. In all of the drawings, the same constituents are given the same reference numerals, and the description thereof will be appropriately omitted.

### [First Embodiment]

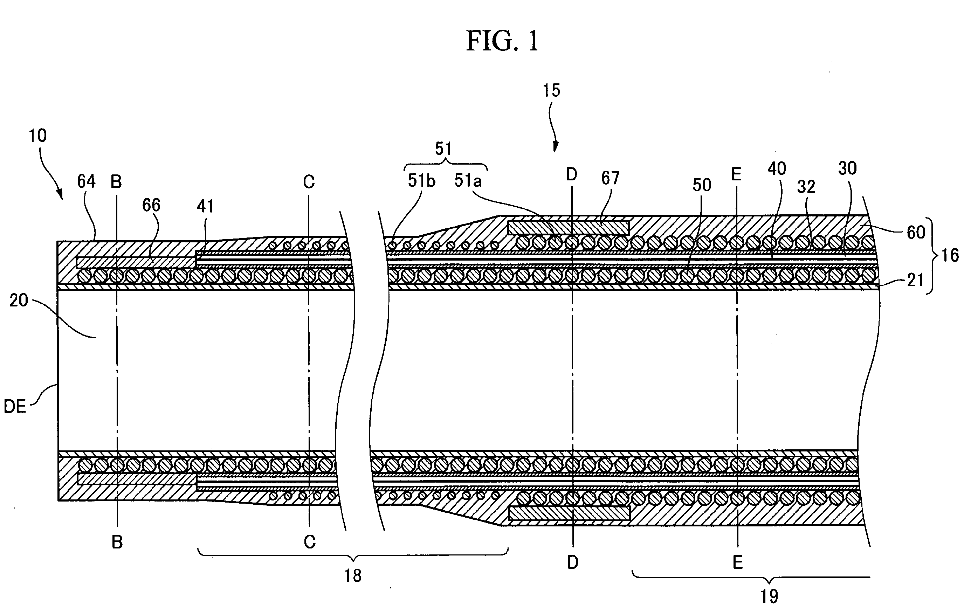

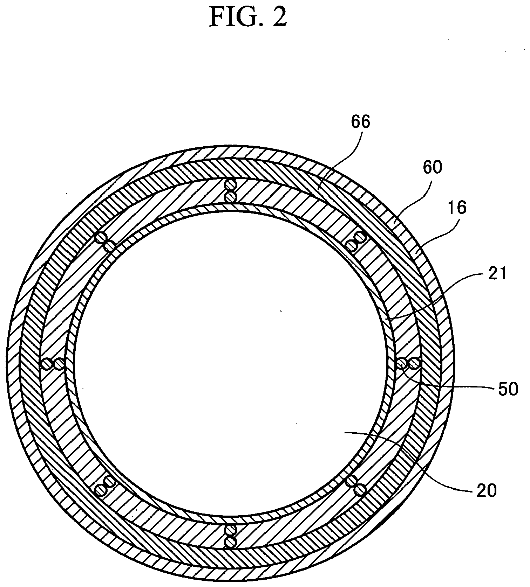

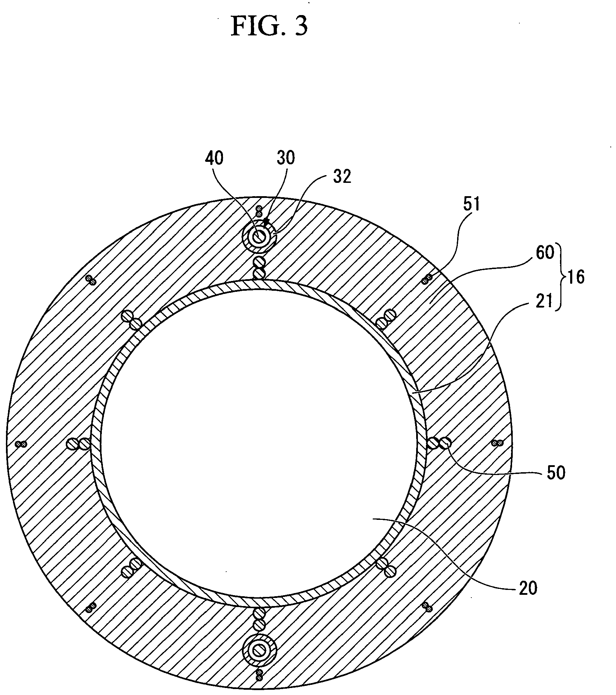

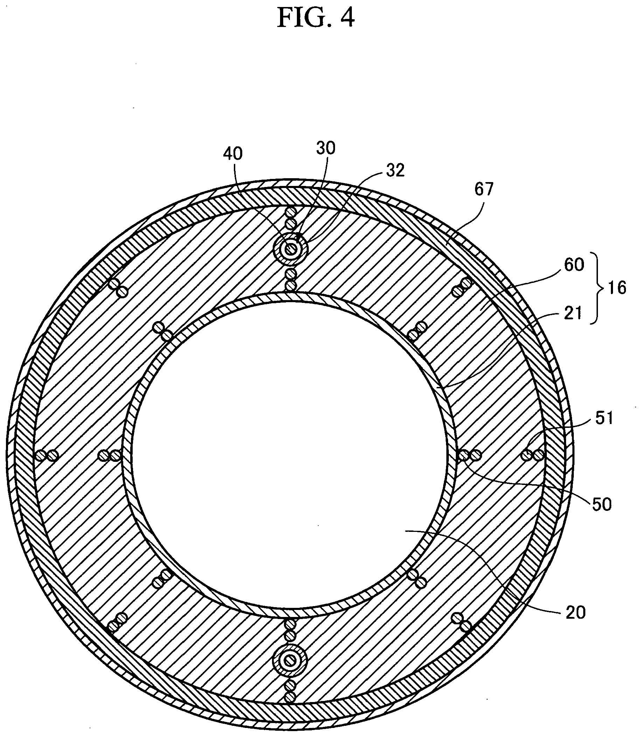

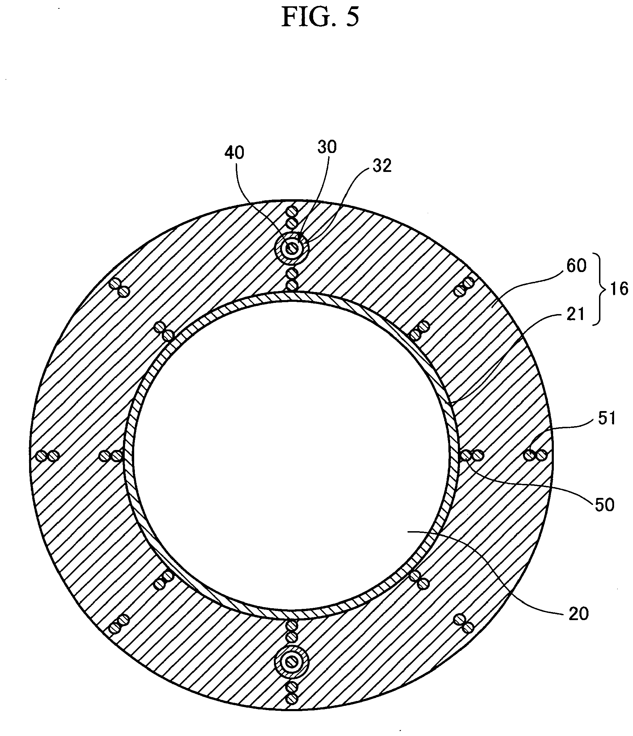

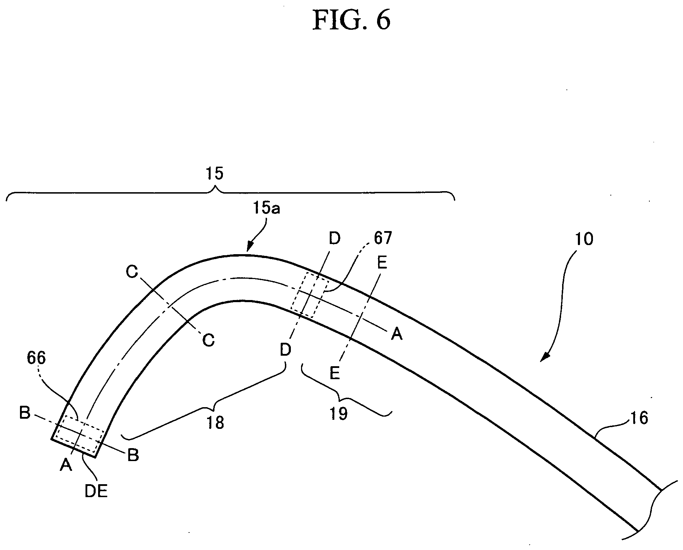

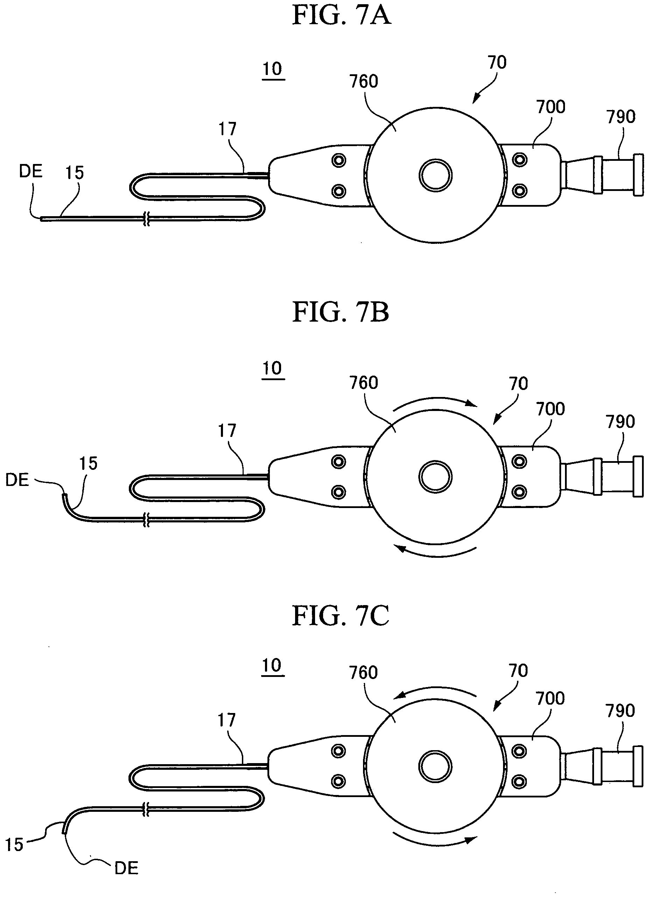

FIG. 1 is a longitudinal cross-sectional view schematically showing a distal portion of a catheter 10 as a medical device according to the embodiment. FIG. 1 is a cross-sectional view taken along A-A line of FIG. 6. FIG. 2 is a horizontal cross-sectional view schematically showing the catheter 10 taken along B-B line of FIG. 1. FIG. 3 is a horizontal cross-sectional view schematically showing the catheter 10 taken along C-C line of FIG. 1. FIG. 4 is a horizontal cross-sectional view schematically showing the catheter 10 taken along D-D line of FIG. 1. FIG. 5 is a horizontal cross-sectional view schematically showing the catheter 10 taken along E-E line of FIG. 1. FIG. 6 is a plan view schematically showing a distal portion of the catheter 10 and a portion in the vicinity thereof. FIG. 7 is a plan view schematically showing the catheter 10.

The catheter 10 according to the present embodiment includes a medical device body (sheath 16); a sub-lumen (for example, sub-lumen 30) which is disposed inside the medical device body so as to extend along a longitudinal direction of the medical device body; an operation wire 40 which is inserted in the sub-lumen and extends along the longitudinal direction of the medical device body; and a first reinforcing layer 50 and a second reinforcing layer 51. The first reinforcing layer 50 and the second reinforcing layer 51, which are in a tubular shape, are embedded in the medical device body such that the axial directions of the reinforcing layers extend along an axial direction of the medical device body, to reinforce the medical device body. The second reinforcing layer 51 is disposed apart from the first reinforcing layer 50 so as to surround the periphery of the first reinforcing layer 50. That is, the first reinforcing layer 50 is disposed inside the second reinforcing layer 51. The space between the first reinforcing layer 50 and the second reinforcing layer 51 is filled with a part of a resin constituting the medical device body. The sub-lumen is disposed inside the part of the resin constituting the medical device body.

Hereinafter, the details thereof will be described.

The sheath 16 is a body of the catheter 10. The sheath 16 is elongated and flexible, and is inserted into a body cavity.

In the present specification, an area with a predetermined length which includes a distal end (tip end) DE of the catheter 10 (as well as the sheath 16) is called a distal portion (tip end portion) 15 of the catheter 10 (as well as the sheath 16). Similarly, an area with a predetermined length which includes a proximal end (base end) (not shown) of the catheter 10 (as well as the sheath 16) is called a proximal portion (base end portion) 17 (FIG. 7) of the catheter 10 (as well as the sheath 16).

A favorable example of the catheter 10 is an intravascular catheter in which the sheath 16 is used by being inserted into a blood vessel. More specifically, a favorable example of the sheath 16 is for the sheath 16 to be formed so as to have a dimension capable of entering any eight sub-segments of the liver.

As shown in FIGS. 1 and 3 to 5, a main lumen 20 and the sub-lumen 30 are formed inside the sheath 16. The main lumen 20 and the sub-lumen 30 extend along a longitudinal direction (horizontal direction in FIG. 1) of (catheter 10) of the sheath 16.

The catheter 10 has, for example, a plurality of sub-lumens 30.

The diameter of each sub-lumen 30 is smaller than that of the main lumen 20. In other words, the main lumen 20, which has a larger diameter than that of the sub-lumen 30, is formed in the sheath 16 along the longitudinal direction of the sheath 16.

The sub-lumen 30 extends from the proximal portion of the sheath 16 to the vicinity of a first marker 66 (FIG. 1) to be described later.

Examples of the horizontal cross-sectional shape of the sub-lumen 30 include a circular shape, an oval shape, an irregular shape (special shape) (to be described later in detail), an elliptical shape, or an ovoid shape.

As shown in FIGS. 3 to 5, in the horizontal cross-section of sheath 16, the sub-lumens 30, and the main lumen 20 and the sub-lumens 30 are independently disposed apart from each other.

The main lumen 20 is disposed, for example, at the center of the horizontal cross-section (cross-section orthogonal to the longitudinal direction) of the sheath 16.

The plurality of sub-lumens 30 are disposed by, for example, being dispersed around the main lumen 20. In the case of the present embodiment, for example, the number of sub-lumens 30 is two, and the sub-lumens 30 are disposed around the main lumen 20 at 180 degrees intervals.

The operation wire 40 is inserted into the sub-lumen 30 and extends along the longitudinal direction of the sub-lumen 30. That is, the operation wire 40 is embedded in the sheath 16 and extends along the longitudinal direction of the sheath 16. In the case of the present embodiment, the catheter 10 has, for example, two operation wires 40.

The operation wire 40 is movable in the longitudinal direction of the sub-lumen 30 relative to the sub-lumen 30, by sliding the peripheral wall of the sub-lumen 30. That is, the operation wire 40 is slidable in the longitudinal direction of the sub-lumen 30.

The operation wire 40 may be formed of a single wire, or be a stranded wire which is formed by twisting a plurality of thin wires.

The number of thin wires constituting the one stranded wire is not particularly limited, but is preferably greater than or equal to three. A favorable example of the number of thin wires is seven or three.

In the case where the number of thin wires constituting the operation wire 40 is three, three thin wires are point-symmetrically disposed in the horizontal cross-section. In the case where the number of thin wires constituting the operation wire 40 is seven, seven thin wires are point-symmetrically disposed in a honeycomb shape in the horizontal cross-section.

The external dimension (diameter of a circumscribed circle of the stranded wire) of the operation wire 40 can be set to, for example, 25 µm to 55 µm.

As materials of the operation wire 40 (or the thin wires constituting the operation wire 40), it is possible to use polymer fibers such as polyparaphenylene benzobisoxazole (PBO), polyetheretherketone (PEEK), polyphenylene sulfide (PPS), polybutylene terephthalate (PBT), polyimide (PI), or polytetrafluoroethylene (PTFE), or boron fibers; in addition to flexible metallic wires such as low carbon steel (piano wires), stainless steel (SUS), titanium or titanium alloy, or tungsten.

The outer diameter dimension of the operation wire 40 (for example, diameter of a circumscribed circle of the stranded wire) can be set to, for example, 25 µm to 55

As will be described later, a tip end portion 41 of the operation wire 40 is fixed to a distal portion 15 of the sheath 16. The sheath 16 can be bent in an intersecting direction with respect to the axial direction of the sheath 16, through a pulling operation with respect to the operation wire 40.

Here, FIG. 6 shows a state in which the distal portion 15 of the sheath 16 is bent through the pulling operation with respect to the operation wire 40.

As shown in FIG. 6, in the distal portion 15 of the sheath 16, a portion (hereinafter, referred to as a maximum bending portion 15a), in which the curvature when performing the pulling operation becomes maximum, is positioned, for example, between the first marker 66 and a second marker 67 to be described later, and nearer to the second marker 67 than the first marker 66. The curvature referred to herein is a curvature of bending of the sheath 16 in an intersecting direction with respect to the axial direction. However, the maximum bending portion 15a may be positioned nearer to the first marker 66 than the second marker 67.

Examples of the structure of the sub-lumen 30 include the following two structures.

In a first structure, as shown in FIGS. 1 and 3 to 5, a resin hollow tube 32 which is previously formed is embedded in an outer layer 60 (to be described later) along the longitudinal direction of the sheath 16, and the lumen of the hollow tube 32 is set to the sub-lumen 30. That is, in this structure, the sub-lumen 30 is formed by the lumen of the resin hollow tube 32 which is embedded in the sheath 16.

The hollow tube 32 can be formed of, for example, a thermoplastic resin. Examples of the thermoplastic resin include low-friction resins such as polytetrafluoroethylene (PTFE) and polyetheretherketone (PEEK).

In a second structure, an elongated hollow is formed in the outer layer 60 (to be described later) along the longitudinal direction of the sheath 16 and the hollow is set to the sub-lumen 30.

More specifically, the sheath 16 has, for example, an inner layer 21; the outer layer 60, which is formed by being stacked around the inner layer 21; and a coating layer 64 which is formed around the outer layer 60.

The inner layer 21 is formed of a tubular resin material. The main lumen 20 is formed at the center of the inner layer 21.

The outer layer 60 is formed of a resin material which is the same or different to the inner layer 21. The sub-lumen 30 is formed inside the outer layer 60.

Examples of the material of the inner layer 21 include a fluoropolymer material. Specific examples of the thermoplastic fluoropolymer material include polytetrafluoroethylene (PTFE), polyvinylidene fluoride (PVDF), or perfluoroalkoxy fluorine resin (PFA).

The delivery property when supplying a patient with a contrast agent, a liquid medicine, a device (to be described later), or the like through the main lumen 20 by forming the inner layer 21 with such a fluorine resin, improves.

Examples of the material of the outer layer 60 include a thermoplastic polymer. Examples of the thermoplastic polymer include polyimide (PI), polyamide-imide (PAI), polyethylene terephthalate (PET), polyethylene (PE), polyamide (PA), polyamide elastomer (PAE), nylon elastomer, polyurethane (PU), ethylene-vinyl acetate (EVA) resins, polyvinyl chloride (PVC), or polypropylene (PP).

The sheath 16 is formed of, for example, a resin material. More specifically, the sheath 16 has a hollow resin layer which includes the above-described outer layer 60 and inner layer 21 which are formed of resin materials. The first reinforcing layer 50 and the second reinforcing layer 51, which are to be described later, are embedded in this resin layer.

The resin material constituting the sheath 16 may include an inorganic filler. For example, as the resin material constituting the outer layer 60 occupying most of the thickness of the sheath 16, it is possible to use a resin material including an inorganic filler.

Examples of such an inorganic filler include barium sulfate or bismuth subcarbonate. The X-ray contrast property is improved by mixing such an inorganic filler into the outer layer 60.

The coating layer 64 constitutes the outermost layer of the catheter 10, and is formed of a hydrophilic material. The coating layer 64 may be formed only in an area over a partial length of the distal portion 15 of the sheath 16, or may be formed over the entire length of the sheath 16.

The coating layer 64 is made to have hydrophilicity by being molded with a hydrophilic resin material such as a maleic anhydride-based polymer such as polyvinyl alcohol (PVA) or a copolymer thereof, or polyvinyl pyrrolidone. The coating layer 64 may be formed by making at least the outer surface of the outer layer 60 hydrophilic by subjecting the outer surface of the outer layer 60 to a lubrication treatment.

The first reinforcing layer 50 and the second reinforcing layer 51 are embedded in the sheath 16 such that the axial directions of the reinforcing layers extend along the axial direction of the sheath 16 (for example, the axial directions of the reinforcing layers become parallel to the axial direction of the sheath 16), to reinforce the sheath 16.

The first reinforcing layer 50 is disposed inside the second reinforcing layer 51. That is, two reinforcing layers (first reinforcing layer 50 and second reinforcing layer 51) are embedded in the sheath 16, and the second reinforcing layer 51 is disposed apart from the first reinforcing layer 50 over the entire peripheral direction and the longitudinal direction of the second reinforcing layer 51 so as to surround the periphery of the first reinforcing layer 50.

The first reinforcing layer 50 is disposed further on the radially inner side of the sheath 16 than an area where the hollow tube 32 is disposed in the sheath 16 (alternately, it can also be regarded as an area where the sub-lumen 30 is disposed, or an area where the operation wire 40 is disposed). The second reinforcing layer 51 is disposed further on the radially outside of the sheath 16 than the area where the hollow tube 32 is disposed in the sheath 16. Accordingly, the hollow tube 32, the sub-lumen 30, and the operation wire 40 are protected by the first reinforcing layer 50 positioned inside and the second reinforcing layer 51 positioned outside.

The hollow tube 32, the sub-lumen 30, and the operation wire 40 are guided to an area further on the distal side than the second marker 67 from an area further on the proximal side than the second marker 67 through a layer between the first reinforcing layer 50 and the second reinforcing layer 51 in the sheath 16.

For example, the first reinforcing layer 50 and the second reinforcing layer 51 are disposed coaxially with the sheath 16. The first reinforcing layer 50 and the second reinforcing layer 51 are disposed, for example, around the inner layer 21. The first reinforcing layer 50 and the second reinforcing layer 51 are, for example, embedded in the outer layer 60.

The space between the first reinforcing layer 50 and the second reinforcing layer 51 is filled with a part of a resin constituting the sheath 16 (for example, the outer layer 60 of the sheath 16) (the part of the resin constituting the sheath is interposed between the first reinforcing layer 50 and the second reinforcing layer 51). The hollow tube 32 is disposed inside the part of the resin constituting the sheath 16 (for example, the outer layer 60 of the sheath 16), between the first reinforcing layer 50 and the second reinforcing layer 51.

Another part of the resin constituting the sheath 16 (for example, the outer layer 60 of the sheath 16) is impregnated in the first reinforcing layer 50 and the second reinforcing layer 51.

Still another part of the resin constituting the sheath 16 (for example, the outer layer 60 of the sheath 16) covers the periphery of the second reinforcing layer 51.

The catheter 10 further has the first marker 66 and the second marker 67. The first marker 66 and the second marker 67 are provided in the distal portion 15 of the sheath 16. The second marker 67 is provided in a portion further on the proximal side of the sheath 16 than the first marker 66. The tip end portion 41 (FIG. 2) of the operation wire 40 is fixed to a portion further on the distal end DE (FIGS. 1 and 2) side than the second marker 67 in the distal portion 15 of the catheter 10.

The first marker 66 and the second marker 67 are formed of a material (radiopaque material) through which a certain radiation such as an X-ray is opaque. The material through which a certain radiation is opaque does not mean a material through which a certain radiation is not opaque at all, but means an opaque material with respect to a certain radiation, that is, a material which has a favorable contrast property when the material is irradiated with a certain radiation. Specifically, the first marker 66 and the second marker 67 are formed of, for example, a metallic material such as platinum.

Each of the first marker 66 and the second marker 67 is formed in, for example, an annular (ring) shape, and is fixed to the sheath 16 in a coaxial arrangement with the sheath 16. Each of the first marker 66 and the second marker 67 is provided, for example, around the main lumen 20, on the first reinforcing layer 50 or the second reinforcing layer 51, and inside the outer layer 60.

The first marker 66 is disposed, for example, further on the radially outside of the sheath 16 than an area where the first reinforcing layer 50 is disposed in the sheath 16. That is, the first marker 66 is disposed so as to surround the periphery of the first reinforcing layer 50. More specifically, the inner peripheral surface of the first marker 66 comes into contact with, for example, the first reinforcing layer 50 (FIG. 2).

The second marker 67 is disposed, for example, further on the radially outside of the sheath 16 than an area where the second reinforcing layer 51 is disposed in the sheath 16. That is, the second marker 67 is disposed so as to surround the periphery of the second reinforcing layer 51. More specifically, the inner peripheral surface of the second marker 67 comes into contact with, for example, the second reinforcing layer 51 (FIG. 4).

The first marker 66 is, for example, caulked and fixed to the periphery in the vicinity of the distal portion of the first reinforcing layer 50. The second marker 67 is, for example, caulked and fixed to the periphery in the vicinity of a proximal side portion 51a of the second reinforcing layer 51.

The second marker 67 is formed so as to have a diameter larger than that of the first marker 66. That is, the dimension of the second marker 67 in the radial direction of the sheath 16 is larger than that of the first marker 66 in the radial direction of the sheath 16.

As described above, the second marker 67 is formed in an annular shape using a metallic material such as platinum. For this reason, an area where the second marker 67 is disposed in the catheter 10 is reinforced by the second marker 67. Moreover, the bending rigidity of the area where the second marker 67 is disposed in the catheter 10 is larger than that of areas (first adjacent area 18 and second adjacent area 19) which are adjacent to the distal side and the proximal side of the area where the second marker 67 is disposed in the catheter 10. With this configuration, it is possible to easily realize a structure in which the portion in the vicinity of the second marker 67 in the catheter 10 becomes the maximum bending portion 15a.

The tip end portion 41 of the operation wire 40 (FIG. 1) is fixed to the distal portion 15 of the catheter 10. More specifically, the tip end portion 41 of the operation wire 40 is fixed to the portion further on the distal side than the second marker 67 in the catheter 10. More specifically, the tip end portion 41 of the operation wire 40 is, for example, fixed to the first marker 66 (for example, fixed to the proximal portion of the first marker 66). Alternately, the tip end portion 41 of the operation wire 40 is fixed to a portion in the vicinity of the first marker 66 in the sheath 16 (to a portion closer to the first marker 66 than the second marker 67 in the distal portion 15 of the sheath 16).

The mode of fixing tip end portion 41 of the operation wire 40 to the distal portion 15 is not particularly limited. For example, the tip end portion 41 may be either welded or fastened to the first marker 66; welded to the distal portion 15 of the sheath 16; or bonded and fixed to the first marker 66 or the distal portion 15 of the sheath 16 using an adhesive.

At least one of the first reinforcing layer 50 and the second reinforcing layer 51 is disposed over an area further on the distal side than the area where the second marker 67 is disposed in the catheter 10, from an area further on the proximal side than the area where the second marker 67 is disposed in the catheter 10.

In the case of the present embodiment, each of the first reinforcing layer 50 and the second reinforcing layer 51 is disposed over the area further on the distal side than the area where the second marker 67 is disposed in the catheter 10, from the area further on the proximal side than the area where the second marker 67 is disposed in the catheter 10.

The second reinforcing layer 51 has the proximal side portion 51a; and a distal side portion 51b which is adjacent to the distal side of the proximal side portion 51a.

The distal side portion 51b is disposed, for example, in the portion further on the distal side than the second marker 67 in the catheter 10.

The proximal side portion 51a is disposed, for example, in a portion further on the proximal side than the area where the second marker 67 is disposed, and the second marker 67.

The bending rigidity of the distal side portion 51b in a direction intersecting with the axial direction of the sheath 16 is smaller than that of the proximal side portion 51a in the direction intersecting with the axial direction of the sheath. That is, the portion (distal side portion 51b) further on the distal side than the second marker 67 in the second reinforcing layer 51 has a smaller bending rigidity in the direction intersecting with the axial direction of the sheath 16 compared to that of the portion (for example, a part of the proximal side portion 51a) further on the proximal side than the second marker 67 in the second reinforcing layer 51.

Specifically, for example, the outer diameters of wires constituting the distal side portion 51b is set to be smaller than those of wires constituting the proximal side portion 51a. Accordingly, the bending rigidity of the distal side portion 51b is set to be smaller than that of the proximal side portion 51a.

For example, the outer diameter of the portion further on the distal side than the second marker 67 in the catheter 10 is set to be smaller compared to that of the portion further on the proximal side than the second marker 67 in the catheter 10.

For example, the outer diameters of the wires constituting the proximal side portion 51a and the outer diameters of wires constituting the first reinforcing layer 50 are set to be equal to each other (for example, the same as each other).

The distal end of the first reinforcing layer 50 reaches, for example, an area where the first marker 66 is disposed. That is, the distal end of the first reinforcing layer 50 is positioned further on the distal end DE of the sheath 16 than the proximal end of the area where the first marker 66 is disposed, and the first reinforcing layer 50 also exists in the area where the first marker 66 is disposed in the longitudinal direction of the catheter 10. Specifically, for example, the distal end of the first reinforcing layer 50 is positioned near the distal end of the first marker 66.

In contrast, the distal end of the second reinforcing layer 51 is positioned further on the proximal side of the catheter 10 than the area where the first marker 66 is disposed. That is, for example, the second reinforcing layer 51 does not exist in the area where the first marker 66 is disposed in the longitudinal direction of the catheter 10. Specifically, for example, the distal end of the second reinforcing layer 51 is positioned between the first marker 66 and the second marker 67 and nearer to the first marker 66 than the second marker 67.

In this manner, the distal end of at least one of the first reinforcing layer 50 and the second reinforcing layer 51 reaches the area where the first marker 66 is disposed. Accordingly, it is possible to suppress generation of a kink in the vicinity at the proximal end of the first marker 66.

The distal end of the inner layer 21 is positioned at the distal end DE of the sheath 16. However, the distal end of the inner layer 21 may be positioned further on the proximal side than the distal end DE of the sheath 16. For example, the distal end of the inner layer 21 may be positioned further on the proximal side of the catheter 10 than the area where the first marker 66 is disposed, and by doing this, the catheter 10 can be more flexibly bent in the vicinity of the first marker 66.

In addition, the distal end of the inner layer 21 may be positioned between the first marker 66 and the second marker 67 and nearer to the first marker 66 than the second marker 67.

Alternately, the distal end of the inner layer 21 may be positioned between the first marker 66 and the second marker 67 and nearer to the second marker 67 than the first marker 66.

The proximal end of the first reinforcing layer 50 and the second reinforcing layer 51 is positioned at, for example, the proximal end of the sheath 16. That is, the proximal end of the first reinforcing layer 50 and the second reinforcing layer 51 is positioned, for example, inside a hub 790 to be described later.

Each of the first reinforcing layer 50 and the second reinforcing layer 51 is, for example, a blade layer or a coil. That is, the first reinforcing layer 50 and the second reinforcing layer 51 may be respectively blade layers or coils, or any one of the first reinforcing layer 50 and the second reinforcing layer 51 may be a blade layer and the other one may be a coil. The blade layer is formed in a tubular shape by braiding wires in a mesh shape. The coil is formed in a tubular shape by winding the wires in a spiral shape.

The first reinforcing layer 50 and the second reinforcing layer 51 in the present embodiment is formed by winding wires, which are disposed apart from each other in the peripheral direction, in a spiral shape in a coaxial arrangement with the sheath 16. Eight wires which are apart from each other in the peripheral direction are disposed in FIGS. 2 to 5, but the number of wires is not limited thereto.

It is possible to make the first reinforcing layer 50 or the second reinforcing layer 51 be a mesh-like blade layer by winding the wires, which are disposed apart from each other in the peripheral direction, in a spiral shape in two or more directions different from each other. In addition, it is possible to make the first reinforcing layer 50 or the second reinforcing layer 51 be a coil by winding the wires, which are disposed apart from the peripheral direction, in a spiral shape in an identical direction.

As the material of the wires constituting the first reinforcing layer 50 and the second reinforcing layer 51, for example, metal is preferably used. However, the present invention is not limited to this example, and other materials (for example, a resin outer layer) may be used as long as the materials have rigidity higher than those of the inner layer 21 and the outer layer 60 and have elasticity. Specific examples of the metallic material of the wire include tungsten (W), stainless steel (SUS), nickel-titanium alloy, steel, titanium, or copper alloy. The shape of the cross-section of the wire is not particularly limited, and preferred examples include a rectangular shape or a circular shape.

For example, the first reinforcing layer 50 can be formed of tungsten. In addition, the second reinforcing layer 51 can be formed of, for example, stainless steel. The outer diameters of the wires constituting the first reinforcing layer 50 and the wires constituting the second reinforcing layer 51 can be set to be, for example, the same as each other.

Here, various properties such as a contrast property or rigidity during X-ray photography vary depending on the type of the metallic material. For this reason, it is possible to finely adjust the various properties such as the contrast property or the rigidity by making the material of the first reinforcing layer 50 and the material of the second reinforcing layer 51 different from each other.

For example, the first reinforcing layer 50 can be set to a blade layer. In addition, for example, the proximal side portion 51a of the second reinforcing layer 51 can be set to a blade layer and the distal side portion 51b of the second reinforcing layer 51 can be set to a coil or a blade layer.

For example, when the first reinforcing layer 50 is regarded as a tube, the outer peripheral surface of the first reinforcing layer 50 comes into contact with the hollow tube 32. Similarly, when the second reinforcing layer 51 is regarded as a tube, the inner peripheral surface of the second reinforcing layer 51 comes into contact with the hollow tube 32. That is, the outer peripheral surface of the first reinforcing layer 50 and the inner peripheral surface of the second reinforcing layer 51 come into contact with the hollow tube 32. In FIGS. 3 to 5, the outer peripheral surface of the first reinforcing layer 50 and the hollow tube 32, or the inner peripheral surface of the second reinforcing layer 51 and the hollow tube 32 are shown in a state where they are not in contact with each other, but are preferably brought into contact with each other. In consideration of the actual embodiment, the invention of the present application will be described by interpreting them as being brought into contact with each other in FIGS. 3 to 5.

Furthermore, when the first reinforcing layer 50 is regarded as a tube, the inner peripheral surface of the first reinforcing layer 50 comes into contact with the outer peripheral surface of the inner layer 21.

In the case where the first reinforcing layer 50 is a blade layer or a coil, the pitch angles of the wires constituting the first reinforcing layer 50 can be made to become gradually smaller from the proximal side to the distal side thereof.

Similarly, the pitch angles of the wires constituting the second reinforcing layer 51 can also be made to become gradually smaller from the proximal side to the distal side thereof.

By doing this, it is possible to make the bending rigidity of the distal portion 15 of the catheter 10 become gradually smaller toward the distal end DE side.

Here, a representative dimension of each constituent of the catheter 10 of the present embodiment will be described.

The radius of the main lumen 20 can be set to about 200 µm to 300 µm, the thickness of the inner layer 21 can be set to about 5 µm to 30 µm, the thickness of the outer layer 60 can be set to about 10 µm to 200 µm, the inner diameter of the first reinforcing layer 50 can be set to about 410 µm to 660 µm, the outer diameter of the first reinforcing layer 50 can be set to about 450 µm to 740 µm, the inner diameter of the second reinforcing layer 51 can be set to about 560 µm to 920 µm, and the outer diameter of the second reinforcing layer 51 can be set to about 600 µm to 940µm.

The inner diameter of the first marker 66 can be set to about 450 µm to 740 µm, the outer diameter of the first marker 66 can be set to about 490 µm to 820 µm, the inner diameter of the second marker 67 can be set to about 600 µm to 940 µm, the outer diameter of the second marker 67 can be set to about 640 µm to 980 µm, the length of the first marker 66 in the longitudinal direction of the sheath 16 can be set to about 0.3 mm to 2.0 mm, and the length of the second marker 67 in the longitudinal direction of the sheath 16 can be set to about 0.3 mm to 2.0 mm.

The radius (distance) from the axial center of the catheter 10 to the center of the sub-lumen 30 is set to about 300 µm to 450 µm, and the inner diameter (diameter) of the sub-lumen 30 is set to 40 µm to 100 µm. The thickness of the operation wire 40 is set to about 30 µm to 60

The outermost diameter (radius) of the catheter 10 is about 350 µm to 490 µm, that is, the outer diameter thereof is less than 1 mm. Accordingly, the catheter 10 of the present embodiment can be inserted into a blood vessel such as a celiac artery.

The sub-lumen 30 is open at least on the proximal side of the catheter 10. The base end portion of each operation wire 40 protrudes on the proximal side from the opening of the sub-lumen 30.

As shown in FIG. 7, the catheter 10 has an operation portion 70 which is provided in a base end portion of the sheath 16. The operation portion 70 constitutes an operation mechanism for performing a bending operation of the distal portion 15 of the sheath 16 together with the operation wire 40 (FIGS. 1 and 3 to 5).

The operation portion 70 has, for example, a body case 700, and a foil operation portion 760 which is provided so as to be rotatable with respect to the body case 700.

The base end portion of the sheath 16 is guided into the body case 700. A rear end portion of the body case 700 is attached with a hub 790. The base end of the sheath 16 is fixed to a front end portion of the hub 790.

The hub 790 is a tubular body in which is a hollow penetrating the front and the rear of the hub 790 is formed. The hollow of the hub 790 communicates with the main lumen 20 of the sheath 16.

The hub 790 is designed such that an injector (syringe), which is not shown in the drawing, can be inserted into the hub from the rear side. It is possible to supply a liquid such as a liquid medicine to the distal end DE of the sheath 16 through the main lumen 20 and to supply the liquid into a body cavity of a patient from a tip end of the sheath 16, by injecting the liquid into the hub 790 using the injector. That is, it is possible to supply a liquid to the distal end from the proximal end of the sheath through the main lumen 20, which is open in the distal end DE of the sheath 16. The supply substance which is supplied to the distal end from the proximal end of the sheath 16 through the main lumen 20 is not limited to a liquid. For example, it is possible to supply a device such as an embolic coil, beads (spherical embolic substance), and n-butyl-2-cianoacrylate (NBCA), which is used as an instant adhesive, to the distal end from the proximal end of the sheath 16 through the main lumen 20.

For example, the operation wire 40 and the hollow tube 32 are branched from the sheath 16 (area excluding the operation wire 40 and the hollow tube 32 in the sheath 16) in the front end portion of the body case 700.

The base end portion of the hollow tube 32 is open and the base end portion of the operation wire 40 protrudes on the proximal side from the opening of the base end portion of the hollow tube 32.

The base end portion of each operation wire 40 directly or indirectly communicates with the foil operation portion 760. It is possible to bend the distal portion 15 (distal portion 15 of the sheath 16) of the catheter 10 by individually pulling each operation wire 40 to the base end side, through a rotating operation of the foil operation portion 760 in any direction.

When an operation is performed in which the foil operation portion 760 is rotated in one direction around a rotary shaft of the foil operation portion as shown in FIG. 7B, one operation wire 40 is pulled to the base end side. Then, a tensile force is applied to the distal portion 15 of the catheter 10 through the one operation wire 40. Accordingly, the distal portion 15 of the sheath 16 is bent toward the sub-lumen 30 side into which the one operation wire 40 has been inserted, based on the axial center of the sheath 16. That is, the distal portion 15 of the sheath 16 is bent in the one direction.

In addition, when an operation is performed in which the foil operation portion 760 is rotated in the other direction around a rotary shaft of the foil operation portion as shown in FIG. 7C, the other operation wire 40 is pulled to the base end side. Then, a tensile force is applied to the distal portion 15 of the catheter 10 through the other operation wire 40. Accordingly, the distal portion 15 of the sheath 16 is bent toward the sub-lumen 30 side into which the other operation wire 40 has been inserted, based on the axial center of the sheath 16. That is, the distal portion 15 of the sheath 16 is bent in the other direction.

Here, the bending of the sheath 16 includes a mode in which the sheath 16 is bent in an "L" shape and a mode in which the sheath 16 is curved in an arched shape.

In this manner, it is possible to bend the distal portion 15 of the catheter 10 in a first direction and a second direction which is opposite to the first direction through an operation of the operation portion 70 and the foil operation portion 760 by selectively pulling the two operation wires 40. The first direction and the second direction are mutually included on an identical plane.

It is possible to freely control the direction of the distal end DE of the catheter 10 by combining a torque operation, in which the entirety of the catheter 10 is axially rotated, and the pulling operation.

Furthermore, it is possible to control the bending amount of the distal portion 15 of the catheter 10 by controlling the pulling amount of the operation wire 40.

For this reason, it is possible to make the catheter 10 of the present embodiment advance to, for example, body cavities such as branched blood vessels in a desired direction.

That is, it is possible to change the advancing direction to the body cavities by performing an operation in which the distal portion 15 is bent.

According to the above-described first embodiment, the second reinforcing layer 51 is disposed apart from the first reinforcing layer 50 so as to surround the periphery of the first reinforcing layer 50; the space between the first reinforcing layer 50 and the second reinforcing layer 51 is filled with a part of a resin constituting the sheath 16; and the sub-lumen 30 is disposed inside the part of the resin constituting the sheath 16. Moreover, the operation wire 40 is inserted into the sub-lumen 30 and extends along the longitudinal direction of the sheath 16. Since the operation wire 40 is inserted into the sub-lumen 30, it is possible to easily move the operation wire in the longitudinal direction of the sub-lumen 30 relative to the sub-lumen 30, by sliding with respect to the peripheral wall of the sub-lumen 30. Moreover, the sub-lumen 30 and the operation wire 40 are protected by the first reinforcing layer 50 positioned inside and the second reinforcing layer 51 positioned outside. Accordingly, the peripheries of the sub-lumen 30 and the operation wire 40 are sufficiently reinforced. Specifically, the hollow tube 32 which includes the sub-lumen 30 and the operation wire 40 is protected by the first reinforcing layer 50 positioned inside and the second reinforcing layer 51 positioned outside.

In this manner, it is possible to provide the catheter 10 that has a configuration in which the operability of the operation wire 40 is good and the periphery of the operation wire 40 is sufficiently reinforced.

Particularly, it is possible to remarkably improve the slidability of the operation wire 40 in the sub-lumen 30 by making the horizontal cross-sectional shape of the sub-lumen 30 be a smooth shape such as a circular shape, an oval shape, an elliptical shape, or an ovoid shape.

Alternately, it is also possible to remarkably improve the slidability of the operation wire 40 in the sub-lumen 30 by making the horizontal cross-sectional shape of the sub-lumen 30 be a shape in which the sub-lumen is brought into contact with the operation wire, or a special shape in which the sub-lumen is brought into contact with the operation wire. Examples of such a special shape include a shape which has one or a plurality of strings (ribs) which protrude on the central side of the horizontal cross-section of the sub-lumen 30 from the peripheral wall of the sub-lumen 30, and extend in the longitudinal direction of the sub-lumen 30, or a star shape.

It is possible to adequately adjust the thickness of the sheath 16 by forming the sheath 16 (specifically, the outer layer 60) so as to cover the second reinforcing layer 51.

In addition, another part of the resin constituting the sheath 16 is impregnated in the first reinforcing layer 50 and the second reinforcing layer 51. That is, the space between the first reinforcing layer 50 and the second reinforcing layer 51, which are a coil or a mesh-like blade layer, is filled with another part of the resin constituting the sheath 16. Accordingly, high integrity between the sheath 16 and the first reinforcing layer 50 and the second reinforcing layer 51 is obtained. It is possible to further secure the reinforcement of the sheath 16 using the first reinforcing layer 50 and the second reinforcing layer 51.

In addition, the first marker 66 and the second marker 67 are provided in the distal portion 15 of the sheath 16 and the tip end portion 41 of the operation wire 40 is fixed to the portion further on the distal side than the second marker 67. Accordingly, it is possible to easily determine an optimum timing for performing the bending operation of the sheath 16 using the positions of the two markers including the first marker 66 and the second marker 67 as indexes. The tip end portion 41 of the operation wire 40 is fixed to the portion further on the distal side than the second marker 67 in the catheter 10. Accordingly, the portion in the sheath 16 further on the distal side than the second marker is bent by the bending operation.

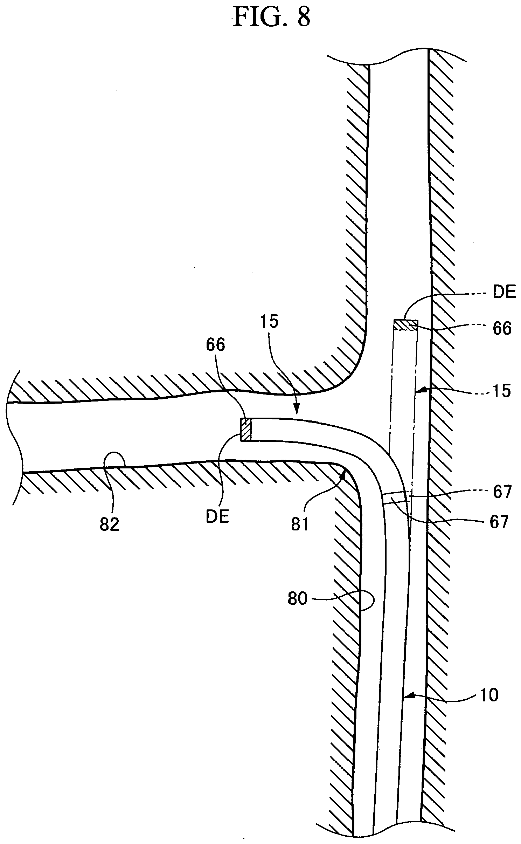

For example, as shown in FIG. 8, it is possible to easily determine the timing for performing the bending operation using a position of the first marker 66, a position of the second marker 67, a position of a branch point 81 of a blood vessel (body cavity) 80, and the like as indexes. Therefore, it is possible to easily advance the distal end DE of the sheath 16 into a desired branch 82 side of the blood vessel 80.

For example, it is possible to instantaneously recognize the distance between the first marker 66 and the branch point 81, the distance between the second marker 67 and the branch point 81, the distance between the first marker 66 and the second marker 67, and the relationship between these distances and the thickness of the blood vessel 80. Therefore, it is possible to easily determine the timing for performing the bending operation of the sheath 16 compared to a case where there is only one marker. Similarly, it is also possible to easily determine a necessary bending amount through the bending operation.

Moreover, at least one of the first reinforcing layer 50 and the second reinforcing layer 51 is disposed over the area where the first marker 66 is disposed, from the area further on the proximal side than the area where the second marker 67 is disposed in the catheter 10. Accordingly, it is possible to reinforce the area between the first marker 66 and the second marker 67 using at least one of the first reinforcing layer 50 and the second reinforcing layer 51, and to suppress generation of a kink or the like of the catheter 10 in the area.

In addition, each of the first reinforcing layer 50 and the second reinforcing layer 51 is disposed over the area further on the distal side than the area where the second marker 67 is disposed in the catheter 10, from the area further on the proximal side than the area where the second marker 67 is disposed in the catheter 10. Accordingly, it is possible to suppress generation of a kink or the like in areas adjacent to the proximal side and the distal side of the second marker 67 in the catheter 10.

In addition, the portion further on the distal side than the second marker 67 in the second reinforcing layer 51 has a smaller bending rigidity in the direction intersecting with the axial direction of the sheath 16 compared to that of the portion further on the proximal side than the second marker 67 in the second reinforcing layer 51. Accordingly, it is possible to reduce the bending rigidity of the portion further on the distal side than the second marker 67 in the catheter 10 and to secure a sufficient bending property.

In addition, the distal end of the second reinforcing layer 51 is positioned further on the proximal side of the sheath 16 than the area where the first marker 66 is disposed. Accordingly, it is possible to suppress the bending rigidity of the catheter 10, in the vicinity of the area where the first marker 66 is arranged, from excessively increasing and to sufficiently secure the bending property of the catheter 10 in the vicinity of the area where the first marker 66 is disposed.

In addition, the distal end of the first reinforcing layer 50 is positioned further on the distal side of the sheath 16 than the proximal end of the area where the first marker 66 is disposed. Accordingly, it is possible to moderately reinforce the area between the area where the second marker 66 is disposed in the sheath 16 and the area where the first marker 66 is disposed and to suppress generation of a kink or the like in the areas.

In addition, the second marker 67 is formed in an annular shape and is disposed around the second reinforcing layer 51, and the inner peripheral surface of the second marker 67 comes into contact with the second reinforcing layer 51. Accordingly, it is possible to support the second marker 67 using the second reinforcing layer 51 and to suppress the outer diameter of the second marker 67 from excessively increasing. Therefore, it is possible to smoothly advance the catheter 10 into the body cavity. Furthermore, it is possible to protect the operation wire 40, the sub-lumen 30, and the hollow tube 32 from the second marker 67 using the second reinforcing layer 51.

In addition, the first marker 66 is formed in an annular shape and is disposed around the first reinforcing layer 50, and the inner peripheral surface of the first marker 66 comes into contact with the first reinforcing layer 50. Accordingly, it is possible to support the first marker 66 using the first reinforcing layer 50 and to suppress the outer diameter of the first marker 66 from excessively increasing. Therefore, it is possible to smoothly advance the catheter 10 into the body cavity.

In addition, since the outer peripheral surface of the first reinforcing layer 50 and the inner peripheral surface of the second reinforcing layer 51 come into contact with the hollow tube 32, it is possible to suppress the outer diameter of the catheter 10 from excessively increasing. Moreover, it is possible to smoothly advance the catheter 10 into the body cavity even with the provision of the first reinforcing layer 50 and the second reinforcing layer 51.

In addition, the operation wire 40 (specifically, the hollow tube 32 which includes the sub-lumen 30 and the operation wire 40) is guided into the area further on the distal side than the second marker 67 in the sheath 16, from the area further on the proximal side than the second marker 67 in the sheath 16, through the layer between the first reinforcing layer 50 and the second reinforcing layer 51 in the sheath 16. Accordingly, it is possible to further protect the operation wire 40 (hollow tube 32 which includes the sub-lumen 30 and the operation wire 40) using the first reinforcing layer 50 and the second reinforcing layer 51 and to suppress interference of the operation wire 40 (hollow tube 32 which includes the sub-lumen 30 and the operation wire 40) with respect to the first reinforcing layer 50 and the second reinforcing layer 51.

In the above, the catheter 10 has been exemplified as a medical device. However, the present invention is also applicable to other medical devices (for example, an endoscope) having a structure in which an elongated medical device body is bent by an operation of pulling the operation wire 40.

In the above, an example has been described in which a sufficient bending property of the portion on the distal side of the catheter 10 is secured due to a smaller bending rigidity of the distal side portion 51b of the second reinforcing layer 51 than that of the proximal side portion 51a. However, a sufficient bending property of the portion on the distal side of the catheter 10 may be secured by a second reinforcing layer 51 which does not have the distal side portion 51b (there is no second reinforcing layer 51 further on the distal side of the second marker 67). As a preferred example, when the second reinforcing layer 51 does not have the distal side portion 51b, the distal end of the second reinforcing layer 51 may be positioned further on the proximal side than the distal end of the second marker 67 and further on the distal side than the proximal end of the second marker 67.

Alternately, a sufficient bending property of the portion on the distal side of the catheter 10 may be secured by the first reinforcing layer 50 existing further on the distal side than the second marker 67.

In the above, an example has been described in which the second reinforcing layer 51 has the proximal side portion 51a and the distal side portion 51b which has a bending rigidity smaller than that of the proximal side portion 51a. However, the bending rigidity of the second reinforcing layer 51 may be constant over the entire length of the second reinforcing layer 51.

In the above, an example has been described in which the first marker 66 is disposed around the first reinforcing layer 50. However, the first marker 66 may be disposed inside the first reinforcing layer 50. Alternately, the proximal end of the second reinforcing layer 51 may reach the area where the first marker 66 is disposed, and the first marker 66 may be disposed around the second reinforcing layer 51.

In the above, an example has been described in which the first reinforcing layer 50 and the second reinforcing layer 51 come into contact with the hollow tube 32. However, a configuration (configuration in which the reinforcing layers are apart from the hollow tube 32) may be employed in which at least one of the first reinforcing layer 50 and the second reinforcing layer 51 does not come into contact with the hollow tube 32.

In the above, an example has been described in which the distal end of the first reinforcing layer 50 is positioned further on the distal side than the proximal end of the first marker 66. However, the distal end of the first reinforcing layer 50 may be positioned further on the proximal side than the proximal end of the first marker 66.

In the above, an example has been described in which the distal end of the second reinforcing layer 51 is positioned further on the proximal side than the proximal end of the first marker 66. However, the distal end of the second reinforcing layer 51 may be positioned further on the distal side than the proximal end of the first marker 66.

In the above, an example has been described in which each of the first reinforcing layer 50 and the second reinforcing layer 51 is positioned over the area further on the distal side than the area where the second marker 67 is disposed, from the area further on the proximal side than the area where the second marker 67 is disposed. However, each of the first reinforcing layer 50 and the second reinforcing layer 51 may be positioned over the area where the second marker 51 is disposed from the area further on the proximal side than the area where the second marker 67 is disposed. That is, the distal end of each of the first reinforcing layer 50 and the second reinforcing layer 51, in particular, the distal end of the second reinforcing layer 51, may be positioned further on the proximal side than the distal end of the second marker 67 and further on the distal side than the proximal end of the second marker 67. In this case, at least a part of the inner peripheral surface of the second marker 67 may come into contact with the second reinforcing layer 51 while the example has been described in the above in which the inner peripheral surface of the second marker 67 comes into contact with the second reinforcing layer 51.

In the above, an example has been described in which the second marker 67 is disposed around the second reinforcing layer 51. However, the second marker 67 may be disposed inside the second reinforcing layer 51 and around the first reinforcing layer 50. In this case, the second marker 67 may be disposed in a layer between the hollow tube 32 and the second reinforcing layer 51 in the sheath 16, or in a layer between the hollow tube 32 and the first reinforcing layer 50 in the sheath 16. In addition, the second marker 67 may be disposed inside the first reinforcing layer 50.

It is unnecessary for each constituent in each form described above to independently exist. A plurality of constituents may be formed as one member; one constituent may be formed as a plurality of members; a certain constituent may be a part of another constituent; and a part of a certain constituent may overlap a part of another constituent.

### Reference Signs List

- 10: catheter
- 15: distal portion
- 15a: maximum bending portion
- 16: sheath
- 17: proximal portion
- 18: first adjacent area
- 19: second adjacent area
- 20: main lumen
- 21: inner layer
- 30: sub-lumen
- 32: hollow tube
- 40: operation wire
- 41: tip end portion
- 50: first reinforcing layer
- 51: second reinforcing layer
- 51a: proximal side portion
- 51b: distal side portion
- 60: outer layer
- 64: coating layer
- 66: first marker
- 67: second marker
- 70: operation portion
- 80: blood vessel (body cavity)
- 81: branch point
- 82: branch
- 700: body case
- 760: foil operation portion
- 790: hub
- DE: distal end

## Claims

1. A medical device, comprising:
an elongated resin medical device body which is flexible and is inserted into a body cavity;
a sub-lumen (30) which is disposed inside the medical device body so as to extend along a longitudinal direction of the medical device body;
an operation wire (40) which is inserted into the sub-lumen (30) and extends along the longitudinal direction of the medical device body, and in which the medical device body is bent through a pulling operation with respect to the operation wire (40);
a first tubular reinforcing layer (50) and a second tubular reinforcing layer (51) which are embedded in the medical device body such that axial directions of the reinforcing layers (50, 51) extend along an axial direction of the medical device body, to reinforce the medical device body;
a first marker (66) which is provided at a distal portion (15) of the medical device body and is formed of a radiopaque material; and
a second marker (67) which is provided further on a proximal side than the first marker (66) in the distal portion (15) of the medical device body and is formed of a radiopaque material,
wherein the second reinforcing layer (51) is disposed apart from the first reinforcing layer (50) so as to surround the periphery of the first reinforcing layer (50),
wherein the space between the first reinforcing layer (50) and the second reinforcing layer (51) is filled with a part of a resin constituting the medical device body,
wherein the sub-lumen (30) is disposed inside the part of the resin,
wherein a tip end portion (41) of the operation wire (40) is fixed further on a distal side than the second marker (67) in the medical device,
wherein at least one of the first reinforcing layer (50) and the second reinforcing layer (51) is disposed over an area where the first marker (66) is disposed,
wherein a distal end of the second reinforcing layer (51) is positioned further on the proximal side of the medical device body than the area where the first marker (66) is disposed, and
wherein a distal end of the first reinforcing layer (50) is positioned further on the distal side of the medical device body than a proximal end of the area where the first marker (66) is disposed.

2. The medical device according to Claim 1,
wherein another part of the resin constituting the medical device body is impregnated in the first reinforcing layer (50) and the second reinforcing layer (51).

3. The medical device according to Claim 1 or 2,
wherein each of the first reinforcing layer (50) and the second reinforcing layer (51) is disposed over an area where the second marker (67) is disposed in the medical device.

4. The medical device according to any one of Claims 1 to 3,
wherein the second marker (67) is formed in an annular shape and is disposed around the second reinforcing layer (51), and
wherein an inner peripheral surface of the second marker (67) comes into contact with the second reinforcing layer (51).

5. The medical device according to any one of Claims 1 to 4,
wherein a tip end portion (41) of the operation wire (40) is fixed either to the first marker (66), or to a portion closer to the first marker (66) than the second marker (67) in the distal portion (15) of the medical device body.

6. The medical device according to any one of Claims 1 to 5,
wherein each of the first reinforcing layer (50) and the second reinforcing layer (51) is either a blade layer which is formed by braiding wires in a mesh shape, or a coil which is formed by winding the wires in a spiral shape.

7. The medical device according to any one of Claims 1 to 6,
wherein the sub-lumen (30) is formed through a lumen of a resin hollow tube which is embedded in the medical device body.

8. The medical device according to Claim 7,
wherein an outer peripheral surface of the first reinforcing layer (50) and an inner peripheral surface of the second reinforcing layer (51) come into contact with the hollow tube.

9. The medical device according to any one of Claims 1 to 8,
wherein a main lumen (20) which has a diameter larger than the inner diameter of the sub-lumen (30) is formed along a longitudinal direction of the medical device body, in an area further on the radially inside of the medical device body than the area where the first reinforcing layer (50) is disposed, in the medical device body.

10. The medical device according to Claim 9,
wherein the main lumen (20) is open at a distal end of the medical device body, and
wherein a supply substance can be supplied to the distal end from a proximal end of the medical device body through the main lumen (20).

11. The medical device according to any one of Claims 1 to 10,
wherein the horizontal cross-sectional shape of the sub-lumen (30) is in a circular shape, an oval shape, an irregular shape, an elliptical shape, or an ovoid shape.

12. The medical device according to any one of Claims 1 to 11,
wherein the second reinforcing layer (51) is disposed apart from the first reinforcing layer (50) over the entire peripheral direction and the longitudinal direction of the second reinforcing layer (51).

13. The medical device according to any one of Claims 1 to 12,
wherein the medical device is a catheter (10).

## Patentansprüche

1. Medizinische Vorrichtung, umfassend:
einen länglichen medizinischen Vorrichtungskörper aus Harz, welcher flexibel ist und in eine Körperhöhle eingesetzt ist;
ein Sublumen (30), welches innerhalb des medizinischen Vorrichtungskörpers angeordnet ist, um sich entlang einer longitudinalen Richtung des medizinischen Vorrichtungskörpers zu erstrecken;
einen Betätigungsdraht (40), welcher in das Sublumen (30) eingesetzt ist und sich entlang der longitudinalen Richtung des medizinischen Vorrichtungskörpers erstreckt, und wobei der medizinische Vorrichtungskörper durch eine Ziehbetätigung bezüglich des Betätigungsdrahts (40) gekrümmt wird;
eine erste röhrenförmige Verstärkungsschicht (50) und eine zweite röhrenförmige Verstärkungsschicht (51), welche in dem medizinischen Vorrichtungskörper derart eingebettet sind, dass sich axiale Richtungen der Verstärkungsschichten (50, 51) entlang einer axialen Richtung des medizinischen Vorrichtungskörpers zur Verstärkung des medizinischen Vorrichtungskörpers erstrecken;
einen ersten Marker (66), welcher an einem distalen Abschnitt (15) des medizinischen Vorrichtungskörpers bereitgestellt ist und aus einem radiopaken Material gebildet ist; und
einen zweiten Marker (67), welcher weiter auf einer proximalen Seite bereitgestellt ist als der erste Marker (66) in dem distalen Abschnitt (15) des medizinischen Vorrichtungskörpers und aus einem radiopaken Material gebildet ist,
wobei die zweite Verstärkungsschicht (51) getrennt von der ersten Verstärkungsschicht (50) angeordnet ist, um die Peripherie der ersten Verstärkungsschicht (50) zu umgeben,
wobei der Raum zwischen der ersten Verstärkungsschicht (50) und der zweiten Verstärkungsschicht (51) mit einem Teil eines Harzes, das den medizinischen Vorrichtungskörper bildet, gefüllt ist,
wobei das Sublumen (30) innerhalb des Teils des Harzes angeordnet ist,
wobei ein Spitzenendabschnitt (41) des Betätigungsdrahts (40) weiter auf einer distalen Seite befestigt ist als der zweite Marker (67) in der medizinischen Vorrichtung,
wobei mindestens eine der ersten Verstärkungsschicht (50) und der zweiten Verstärkungsschicht (51) über einen Bereich angeordnet ist, wo der erste Marker (66) angeordnet ist,
wobei ein distales Ende der zweiten Verstärkungsschicht (51) weiter auf der proximalen Seite des medizinischen Vorrichtungskörpers positioniert ist als der Bereich, wo der erste Marker (66) angeordnet ist, und
wobei ein distales Ende der ersten Verstärkungsschicht (50) weiter auf der distalen Seite des medizinischen Vorrichtungskörpers positioniert ist als ein proximales Ende des Bereichs, wo der erste Marker (66) angeordnet ist.

2. Medizinische Vorrichtung nach Anspruch 1,
wobei ein anderer Abschnitt des Harzes, das den medizinischen Vorrichtungskörper bildet, in der ersten Verstärkungsschicht (50) und der zweiten Verstärkungsschicht (51) imprägniert ist.

3. Medizinische Vorrichtung nach Anspruch 1 oder 2,
wobei die erste Verstärkungsschicht (50) und die zweite Verstärkungsschicht (51) jeweils über einen Bereich angeordnet sind, wo der zweite Marker (67) in der medizinischen Vorrichtung angeordnet ist.

4. Medizinische Vorrichtung nach einem der Ansprüche 1 bis 3,
wobei der zweite Marker (67) in einer ringförmigen Form gebildet ist und um die zweite Verstärkungsschicht (51) angeordnet ist, und
wobei eine innere periphere Oberfläche des zweiten Markers (67) in Kontakt mit der zweiten Verstärkungsschicht (51) tritt.

5. Medizinische Vorrichtung nach einem der Ansprüche 1 bis 4,
wobei ein Spitzenendabschnitt (41) des Betätigungsdrahts (40) entweder an dem ersten Marker (66) oder an einem Abschnitt, der dem ersten Marker (66) näher liegt als dem zweiten Marker (67), in dem distalen Abschnitt (15) des medizinischen Vorrichtungskörpers befestigt ist.

6. Medizinische Vorrichtung nach einem der Ansprüche 1 bis 5,
wobei die erste Verstärkungsschicht (50) und die zweite Verstärkungsschicht (51) jeweils entweder eine Lamellenschicht, welche durch Flechten von Drähten in einer Netzform gebildet ist, oder eine Spule, welche durch Wickeln der Drähte in einer Spiralform gebildet ist, sind.

7. Medizinische Vorrichtung nach einem der Ansprüche 1 bis 6,
wobei das Sublumen (30) durch ein Lumen eines Hohlrohres aus Harz gebildet ist, welches in dem medizinischen Vorrichtungskörper eingebettet ist.

8. Medizinische Vorrichtung nach Anspruch 7,
wobei eine äußere periphere Oberfläche der ersten Verstärkungsschicht (50) und eine innere periphere Oberfläche der zweiten Verstärkungsschicht (51) in Kontakt mit dem Hohlrohr treten.

9. Medizinische Vorrichtung nach einem der Ansprüche 1 bis 8,
wobei ein Hauptlumen (20), welches einen Durchmesser aufweist, der größer ist als der Innendurchmesser des Sublumens (30), entlang einer longitudinalen Richtung des medizinischen Vorrichtungskörpers, in einem Bereich, der weiter auf der radialen Innenseite des medizinischen Vorrichtungskörpers liegt als der Bereich, wo die erste Verstärkungsschicht (50) angeordnet ist, in dem medizinischen Vorrichtungskörper gebildet ist.

10. Medizinische Vorrichtung nach Anspruch 9,
wobei das Hauptlumen (20) an einem distalen Ende des medizinischen Vorrichtungskörpers offen ist, und
wobei eine zuzuführende Substanz zu dem distalen Ende von einem proximalen Ende des medizinischen Vorrichtungskörpers durch das Hauptlumen (20) zugeführt werden kann.

11. Medizinische Vorrichtung nach einem der Ansprüche 1 bis 10,
wobei die horizontale Querschnittsform des Sublumens (30) in einer kreisförmigen Form, einer ovalen Form, einer unregelmäßigen Form, einer ellipsenförmigen Form oder einer eiförmigen Form vorliegt.

12. Medizinische Vorrichtung nach einem der Ansprüche 1 bis 11,
wobei die zweite Verstärkungsschicht (51) getrennt von der ersten Verstärkungsschicht (50) über die gesamte periphere Richtung und die longitudinale Richtung der zweiten Verstärkungsschicht (51) angeordnet ist.

13. Medizinische Vorrichtung nach einem der Ansprüche 1 bis 12,
wobei die medizinische Vorrichtung ein Katheter (10) ist.

## Revendications

1. Dispositif médical comprenant:
un corps de dispositif médical en résine de forme allongée, lequel est souple et est inséré dans un cavité corporelle;
une sous-lumière (30) disposée à l'intérieur du corps de dispositif médical de manière à s'étendre dans une direction longitudinale du corps de dispositif médical;
un fil d'opération (40) inséré dans la sous-lumière (30) et qui s'étend dans la direction longitudinale du corps de dispositif médical, et dans lequel le corps de dispositif médical est plié au moyen d'une opération de traction par rapport au fil d'opération (40);
une première couche de renforcement tubulaire (50) et une deuxième couche de renforcement tubulaire (51) intégrées au corps de dispositif médical de sorte que les directions axiales des couches de renforcement (50, 51) s'étendent le long d'une direction axiale du corps de dispositif médical, pour renforcer le corps de dispositif médical;
un premier marqueur (66) prévu à une partie distale (15) du corps de dispositif médical et qui est formé d'un matériau radio-opaque; et
un deuxième marqueur (67) qui est disposé plus loin sur un côté proximal que le premier marqueur (66) dans la partie distale (15) du corps de dispositif médical et est formé d'un matériau radio-opaque,
dans lequel la deuxième couche de renforcement (51) est disposée à distance de la première couche de renforcement (50) de manière à entourer la périphérie de la première couche de renforcement (50);
dans lequel l'espace entre la première couche de renforcement (50) et la deuxième couche de renforcement (51) est remplie d'une partie d'une résine constituant le corps de dispositif médical,
dans lequel la sous-lumière (30) est disposée à l'intérieur de la partie de la résine,
dans lequel une partie terminale d'extrémité (41) du fil d'opération (40) est fixée plus loin sur un côté distal que le deuxième marqueur (67) dans le dispositif médical,
dans lequel au moins l'une de la première couche de renforcement (50) et de la deuxième couche de renforcement (51) est disposée couvrant une zone où le premier marqueur (66) est disposé,
dans lequel une extrémité distale de la deuxième couche de renforcement (51) est positionnée plus loin sur le côté proximal du corps de dispositif médical que la zone où le premier marqueur (66) est disposé, et
dans lequel une extrémité distale de la première couche de renforcement (50) est positionnée plus loin sur le côté distal du corps de dispositif médical qu'une extrémité proximale de la zone où le premier marqueur (66) est disposé.

2. Dispositif médical selon la revendication 1,
dans lequel une autre partie de la résine constituant le corps de dispositif médical est imprégnée dans la première couche de renforcement (50) et la deuxième couche de renforcement (51).

3. Dispositif médical selon la revendication 1 ou 2,
dans lequel chacune de la première couche de renforcement (50) et de la deuxième couche de renforcement (51) est disposée couvrant une zone où le deuxième marqueur (67) est disposé dans le dispositif médical.

4. Dispositif médical selon l'une quelconque des revendications 1 à 3,
dans lequel le deuxième marqueur (67) est usiné dans une forme annulaire et est disposé autour de la deuxième couche de renforcement (51), et
dans lequel une surface périphérique intérieure du deuxième marqueur (67) entre en contact avec la deuxième couche de renforcement (51).

5. Dispositif médical selon l'une quelconque des revendications 1 à 4,
dans lequel une partie terminale d'extrémité (41) du fil d'opération (40) est fixée soit au premier marqueur (66), soit à une partie plus proche du premier marqueur (66) que le deuxième marqueur (67) dans la partie distale (15) du corps de dispositif médical.

6. Dispositif médical selon l'une quelconque des revendications 1 à 5,
dans lequel chacune de la première couche de renforcement (50) et de la deuxième couche de renforcement (51) est soit une couche de lame formée en fils tressés en maille, soit une bobine formée par enroulement des fils en spirale.

7. Dispositif médical selon l'une quelconque des revendications 1 à 6,
dans lequel la sous-lumière (30) est formé par une lumière d'un tube creux de résine intégré au corps de dispositif médical.

8. Dispositif médical selon la revendication 7,
dans lequel une surface périphérique extérieure de la première couche de renforcement (50) et une surface périphérique intérieure de la deuxième couche de renforcement (51) entre en contact avec le tube creux.

9. Dispositif médical selon l'une quelconque des revendications 1 à 8,
dans lequel une lumière principale (20) d'un diamètre plus grand que le diamètre intérieur de la sous-lumière (30) est formé dans une direction longitudinale du corps de dispositif médical, dans une zone plus loin sur l'intérieur radial du corps de dispositif médical que la zone où la première couche de renforcement (50) est disposée, dans le corps de dispositif médical.

10. Dispositif médical selon la revendication 9,
dans lequel la lumière principale (20) est ouverte à une extrémité distale du corps de dispositif médical, et
dans lequel une substance d'alimentation peut être fournie à l'extrémité distale depuis une extrémité proximale du corps de dispositif médical par la lumière principale (20).

11. Dispositif médical selon l'une quelconque des revendications 1 à 10,
dans lequel la forme en coupe horizontale de la sous-lumière (30) est de forme circulaire, de forme ovale, de forme irrégulière, de forme elliptique ou de forme ovoïdale.

12. Dispositif médical selon l'une quelconque des revendications 1 à 11,
dans lequel la deuxième couche de renforcement (51) est disposée à distance de la première couche de renforcement (50) couvrant toute la direction périphérique et la direction longitudinale de la deuxième couche de renforcement (51).

13. Dispositif médical selon l'une quelconque des revendications 1 à 12, dans lequel le dispositif médical est un cathéter (10).
